(19) Europäisches Patentamt
European Patent Office
Office européen des brevets

(11) Veröffentlichungsnummer: **0 352 639 B1**

(12) **EUROPÄISCHE PATENTSCHRIFT**

(45) Veröffentlichungstag der Patentschrift: **19.10.94**

(51) Int. Cl.$^5$: **C07C 217/22**, C07D 295/08, A61K 31/13, A61K 31/395

(21) Anmeldenummer: **89113379.5**

(22) Anmeldetag: **21.07.89**

# (54) p-Hydroxiphenon-Derivate und deren Anwendung.

(30) Priorität: **28.07.88 DE 3825559**

(43) Veröffentlichungstag der Anmeldung:
**31.01.90 Patentblatt 90/05**

(45) Bekanntmachung des Hinweises auf die Patenterteilung:
**19.10.94 Patentblatt 94/42**

(84) Benannte Vertragsstaaten:
**AT BE CH DE FR GB IT LI NL SE**

(56) Entgegenhaltungen:
**DE-A- 2 062 129**
**FR-A- 2 348 189**
**GB-A- 730 922**
**GB-A- 1 022 648**

(73) Patentinhaber: **BASF Aktiengesellschaft**
**Carl-Bosch-Strasse 38**
**D-67063 Ludwigshafen (DE)**

(72) Erfinder: **Lubisch, Wilfried, Dr.**
**Mollstrasse 13**
**D-6800 Mannheim (DE)**
Erfinder: **Raschack, Manfred, Dr.**
**Donnersbergstrasse 7**
**D-6714 Weisenheim am Sand (DE)**
Erfinder: **Von Philipsborn, Gerda, Dr.**
**Naechtstenbacher Weg 35**
**D-6940 Weinheim (DE)**

**EP 0 352 639 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue p-Hydroxiphenon-Derivate, sie enthaltende Arzneimittel und deren Verwendung zur Herstellung von Antiarrhythmika der Klasse III nach Vaughan-Williams.

Die Antiarrhythmika lassen sich auf Grund der Klassifizierung von Vaughan-Williams in 4 Gruppen einteilen:

I. Natrium-Antagonisten,
II. adrenerge β-Rezeptorblocker,
III. Kalium-Kanal-Hemmstoffe,
IV. Calcium-Antagonisten.

Antiarrhythmika der Klasse III weisen häufig den therapeutischen Vorteil auf, gegen sonst therapieresistente Arrhythmien zu wirken, insbesondere in der klinischen Therapie von reentry-Arrhythmien. Dies wurde sowohl beim Amiodaron (Circulation 68 (1), 88-94 (1983)) als auch beim D-Sotalol (Am. Heart J. 109, 949-958 (1985); J. Clin. Pharmacol. 27 (9), 708 (1987)) beschrieben.

p-Hydroxiphenon-Derivate wurden bereits mit unterschiedlicher physiologischer Wirkung beschrieben: als Spasmolytika (DE 26 16 484, DE 11 74 311, Arch. Pharm 1966, 299), Ulkustherapeutika (JP-OS 52/078-858, JP-OS 52/078-856, JP-OS 51/100-050), Amöbizide (FR 5003 M), Vasodilatantien (JP-AS 27177/65, GB 1 022 648, US 3 407 233, DE 2 062 129, JP-AS 40/06903, JP-AS 40/06904, JP-AS 74/021125), und als Fungizide und Antiprotozoön (GB 730 922). Die erfindungsgemäßen neuen p-Hydroxiphenon-Derivate stellen dagegen überraschenderweise Antiarrhythmika der Klasse III dar.

Die Erfindung bezieht sich auf p-Hydroxyphenon-Derivate der Formel I

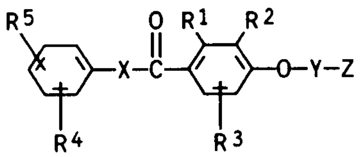

I,

worin

$R^1$, $R^2$ und $R^3$ gleich oder verschieden sind und Methyl, Chlor, Brom sowie H bedeuten, wobei $R^1$, $R^2$ und $R^3$ nicht gleichzeitig H sind,

$R^4$ und $R^5$ gleich oder verschieden sind und H, $C_1$-$C_4$-Alkyl, gerade oder verzweigt, oder $OR^6$ bedeuten, wobei $R^4$ und $R^5$ nicht gleichzeitig H sind,

X -CH=CH-, $-CH_2CH_2$- und $-CH_2$- bedeuten,

Y eine gerade oder verzweigte, gesättigte oder ungesättigte $C_1$-$C_4$-Brücke darstellt und

Z den Rest $NR^6R^7$ darstellt, wobei $R^6$ und $R^7$ unabhängig voneinander Wasserstoff oder $C_{1-4}$-Alkyl bedeuten, oder Z einen 5- oder 6-gliedrigen Ring mit einem Stickstoffatom in 1-Stellung und ggf. zusätzlich einem zweiten N- oder einem O- oder S-Atom bedeutet, wobei das zweite N-Atom durch $R^7$ substituiert ist,

sowie deren physiologisch verträglichen Salze.

Die erfindungsgemäßen Verbindungen können beispielsweise nach folgenden Reaktionsschemata A oder B hergestellt werden:

**Reaktionsschema A:**

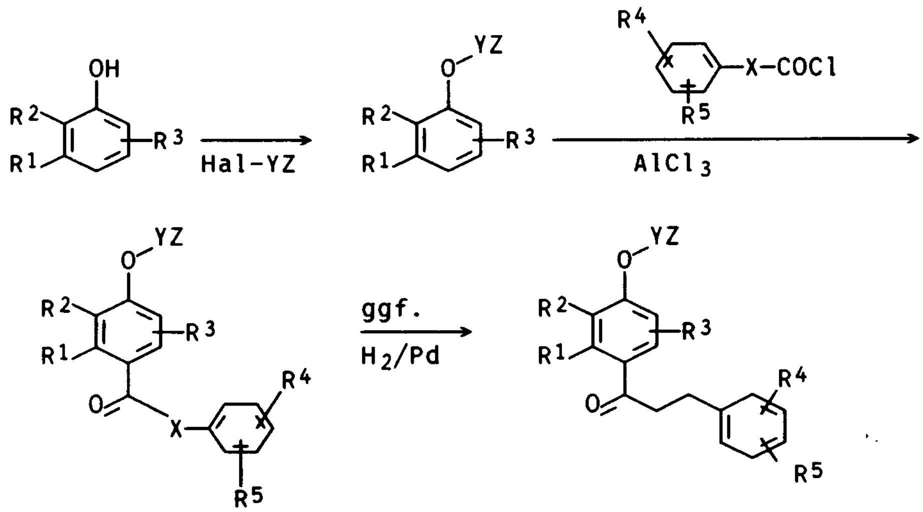


Das Phenol wird in üblicher Weise mit einem Halogenalkylamin lkyliert, wonach eine Friedel-Crafts-Reaktion mit einem Säurechlorid, beispielsweise der Struktur

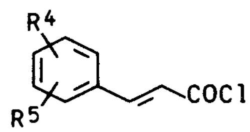

,

zu dem Chalkon der Formel I führt, das ggf. hydriert wird.

**Reaktionsschema B:**

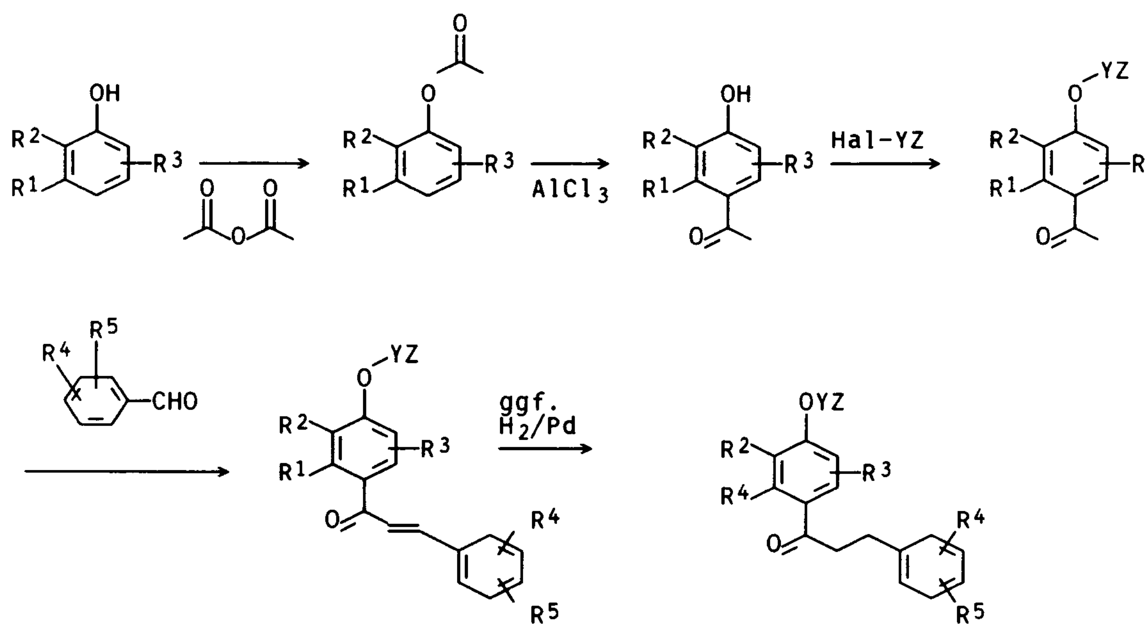


Das Phenol wird mit Essigsäureanhydrid in üblicher Weise verestert. Nach der Fries-Verschiebung in einer $AlCl_3$-Schmelze erfolgt die Phenolalkylierung. Die Kondensation mit einem Aldehyd und die gegebenenfalls gewünschte Hydrierung wird nach üblichen Methoden, die im Houben-Weyl, Methoden der organischen

Chemie, beschrieben sind, ausgeführt.

Die beiden Reaktionsschritte Phenol-Alkylierung und Kondensation können auch in umgekehrter Reihenfolge durchgeführt werden.

Gegebenenfalls werden die so erhaltenen p-Hydroxiphenon-Derivate in das Säureadditionssalz einer physiologisch verträglichen Säure überführt. Eine Zusammenstellung üblicher physiologisch verträglicher Säuren kann aus Fortschritte der Arzneimittelforschung, 1966, Birkhäuser Verlag, Bd. 10, S. 244 bis 285, Deutschland, Schweiz, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohl, wie Methanol, Ethanol oder Propanol, oder einem niederen Keton, wie Aceton, Methylethylketon oder Methylisobutylketon, oder einem Ether, wie Diethylether, Tetrahydrofuran oder Dioxan erhalten. Zur besseren Kristallabscheidung können Mischungen der genannten Lösungsmittel verwendet werden. Darüber hinaus können pharmazeutisch vertretbare wäßrige Lösungen von Säure-Additionsverbindungen der p-Hydroxinphenon-Derivate der Formel I durch Auflösen der freien Basen in einer wäßrigen Säurelösung hergestellt werden.

Die Erfindung betrifft weiter therapeutische Mittel zur topischen und vor allem systemischen Anwendung, die eine Verbindung der Formel (I) neben üblichen Trägerstoffen und/oder sonstigen galenischen Hilfsmitteln als Wirkstoff enthalten, und die Verwendung einer Verbindung der Formel (I) zur Herstellung eines Arzneimittels, insbesondere eines Antiarrhythmikums.

Die neuen Verbindungen besitzen, wie die folgenden Versuchsergebnisse zeigen, eine gute antiarrhythmische Klasse-III-Wirkung:

Als Versuchstiere dienen männliche und weibliche Pirbright-white-Meerschweinchenim Gewicht von 300 bis 500 g. Die Narkose erfolgt mit 1,5 g/kg Urethan i.p. Die Substanzen werden intravenös appliziert. Zur Messung der EKG-Leitungszeiten und der Herzfrequenz wird die II. Extremitätenableitung registriert. Meßgrößen sind die QT- und die PQ-Strecken und die Herzfrequenz. Pro Dosis werden 4 bis 6 Tiere eingesetzt. Kriterium für Klasse-III-Wirkung ist eine Zunahme der QT-Dauer im Vergleich zu den Werten vor Substanzgabe. PQ-Zunahme und starke Herzfrequenzabnahme dienen als Ausschlußkriterien. Aus der linearen Beziehung zwischen log Dosis (mg/kg) der Substanz und der relativen Verlängerung der QT-Dauer (in %) wird die ED 20 % berechnet.

Tabelle 1:

Antiarrhythmische Klasse-III-Wirkung beim Meerschweinchen nach intravenöser Applikation.

| Beispiel Nr. | Verlängerung der QT-Dauer ED 20 % [mg/kg] | rel. Wirkung $\frac{ED_{20}\ (D\text{-}Sotalol)}{ED_{20}\ (Bsp.)}$ |
|---|---|---|
| 1 | 0,74 | 4,9 |
| 2 | 0,76 | 4,7 |
| 3 | 0,65 | 5,5 |
| 4 | 0,89 | 4,0 |
| 10 | 1,31 | 2,7 |
| 12 | 0,74 | 4,9 |
| 13 | 0,67 | 5,4 |
| 15 | 1,5 | 2,4 |
| 17 | 1,69 | 2,1 |
| 18 | 1,5 | 2,4 |
| 20 | 0,73 | 4,9 |
| 24 | 0,95 | 3,8 |
| 25 | 0,79 | 4,6 |
| 27 | 1,2 | 3 |
| 32 | 1,62 | 2,2 |
| D-Sotalol | 3,6 | 1 |

Die erfindungsgemäßen Substanzen (Tabelle 1) sind hinsichtlich QT-Verlängerung stärker wirksam als das bekannte Klasse-III-Antiarrhythmikum D-Sotalol.

Die neuen Substanzen eignen sich daher zur Behandlung sonst therapieresistenter Arrhythmien, insbesondere beseitigen sie ventrikuläre Tachykardien, die nach Myokard-Infarkt auftreten und auf einem "re-entry"-Mechanismus beruhen (Lit. Cardiac Arrhythmia Ed. P. Brugada, H.J.J. Wellens, Futura Publishing Co., Mount Kisko, New York 1987).

Die Herstellung der therapeutischen Mittel oder Zubereitungen erfolgt durch Mischen des Wirkstoffs mit den üblichen flüssigen oder festen Trägerstoffen oder Verdünnungsmitteln und den üblicherweise verwendeten pharmazeutisch-technischen Hilfsstoffen, entsprechend der gewünschten Applikationsart und mit einer zur Anwendung geeigneten Dosierung.

Die Mittel können peroral, parenteral oder topisch verabreicht werden. Derartige Zubereitungen sind beipielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,0001 bis 1 %iger Konzentration, bevorzugt in 0,001 bis 0,1 %iger Konzentration und bei systemischer Anwendung vorzugsweise in einer Einzeldosis von 10 bis 500 mg enthalten und täglich in einer oder mehreren Dosierungen je nach Art und Schwere der Erkrankungen verabreicht werden.

Üblicherweise verwendete pharmazeutisch-technische Hilfsstoffe sind beispielsweise für die lokale Anwendung Alkohole, wie Ethanol, Isopropanol, oxethyliertes Ricinusöl oder oxyethyliertes hydriertes Ricinusöl, Polyacrylsäure, Glycerinmonostearat, Paraffinöl, Vaseline, Wollfett, Polyethylenglykol, Polypropylenglykol, Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol, oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe

toxikologisch unbedenklich und mit den verwendeten Wirkstoffen verträglich sind.

Beispiel 1

3',5'-Dimethyl-4'-(2-(1-pyrrolidinyl)ethoxi)-2-methyl-chalkon-tosylat

46,7 g (0,38 mol) 2,6-Dimethylphenol, 65,0 g (0,38 mol) N-(2-Chlorethyl)pyrrolidinhydrochlorid, 210,1 g (1,52 mol) Kaliumcarbonat und 2 g NaJ wurden in 300 ml Ethylmethylketon 48 h unter Rückfluß gekocht. Das Reaktionsgemisch wurde im Vakuum eingeengt. Der Rückstand wurde zwischen Wasser und Ether verteilt, die organische Phase abgetrennt, mit Wasser gewaschen, getrocknet und mit überschüssiger etherischer Chlorwasserstoff-Lösungversetzt. Das ausgefallene Produkt wurde aus Essigester/Isopropanol (10/1) umkristalliert. Man erhielt 63 g 2-(2,6-Dimethylphenoxi)ethyl-N-pyrrolidinhydrochlorid.

Zu 10 g (39,1 mmol) 2-(2,6-Dimethylphenoxi)ethyl-N-pyrrolidinhydrochlorid und 8,5 g (46,9 mmol) E-(2-Methyl)zimtsäurechlorid, die in 100 ml Methylenchlorid gelöst wurden, gab man portionsweise 10,4 g (78,2 mmol) wasserfreies Aluminiumchlorid. Anschließend wurde alles 1 h auf Rückfluß erwärmt. Das Reaktionsgemisch wurde auf Eis gegossen. Die erhaltene Lösung wurde alkalisiert, die organische Phase abgetrennt, mit Wasser gewaschen und i. Vak. eingeengt. Der Rückstand wurde in wenig heißem Aceton gelöst und mit equimolarer, heißer acetonischer Toluolsulfonsäure-Lösung versetzt. Beim Abkühlen erhielt man 11 g des 3′,5′-Dimethyl-4′-(2-(1-pyrrolidinyl)ethoxi)2-methylchalkon-tosylats.
Schmp.

Analog Beispiel 1 wurden hergestellt:

2. 3′,5′-Dimethyl-4′-(2-(1-piperidinyl)ethoxi)-2-methylchalkon-fumarat
Schmp. 160-163 °C
3. 4′-(2-Diethylamino-ethoxi)-3′,5′-dimethyl-2-methylchalkon-fumarat
Schmp. 128-131 °C
4. 3′,5′-Dimethyl-4′-(3-(1-pyrrolidinyl)propoxi)-2-methylchalkon-fumarat
Schmp. 144-146 °C
5. 4′-(2-Di-n-butylamino-ethoxi)-3′,5′-dimethyl-2-methyl-chalkon-semifumarat
Schmp. 79 °C
6. 2′,6′-Dimethyl-4′-(2-(1-pyrrolidinyl)ethoxi)-2-methylchalkon-semifumarat
Schmp.146-147 °C
7. 4′-(2-(1-Pyrrolidinyl)ethoxi)-2′,3′,5′-trimethyl-2-methylchalkon-fumarat
Schmp. 125-128 °C
8. 3',5'-Dimethyl-4'-(2-(1-pyrrolidinyl)ethoxi)-2-methyl-desoxi-benzoin-oxalat
Schmp. 194-196 °C
9. 3',5'-Dimethyl-4'-(2-(1-pyrrolidinyl)ethoxi)-4-methoxi-desoxi-benzoin-oxalat.
Schmp. 150-152 °C
10. 4'-(2-Diethylaminoethoxi)-3',5'-dimethyl-4-methoxi-desoxi-benzoin
öl

Beispiel 11:

4'-(2-Diethylamino-ethoxi)-2',6'-dimethyl-2-methyl-chalkon-fumarat

206,5 g (1,69 mol) 3,5-Dimethylphenol und 337,3 g (3,30 mol) Acetanhydrid wurden 1 h am Rückfluß gekocht und anschließend über Nacht bei RT gerührt. Man goß auf Wasser und extrahierte mit Methylenchlorid. Die organische Phase wurde mit Natronlauge und Wasser gewaschen, getrocknet und i. Vak. eingeengt. Man erhielt 286 g Essigsäure-3,5-dimethylphenylester.

Zu 246,3 g (1,5 mol) des obigen Produktes wurden portionsweise 206,7 g (1,55 mol) wasserfreies Aluminiumchlorid zugegeben. Man erwärmte für ca. 30 min auf 110 °C und ließ abkühlen. Die glasige Masse wurde in Methylenchlorid gelöst und auf Eis/conc. HCl gegossen. Die organische Phase wurde abgetrennt, mit Wasser gewaschen, getrocknet und i. Vak. eingeengt. Der erhaltene Rückstand wurde aus Ligroin umkristallisiert. Man erhielt 171 g 2,6-Dimethyl-4-hydroxi-acetophenon.

45,15 g (0,275 mol) des obigen Produkts, 37,35 g (0,275 mol) N-(2-Chlorethyl-diethyl)-amin, 75,9 g (0,55 mol) Kaliumcarbonat und 0,5 g 18-krone-6 wurden in 250 ml Ethylmethylketon 2 h unter Rückfluß gekocht. Der Niederschlag wurde abfiltriert und das Filtrat i. Vak. eingeengt. Das so erhaltene Produkt wurde mit Natronlauge aufgeschlämmt und mit Ether extrahiert, der anschließend mit Wasser gewaschen, getrocknet und i. Vak. eingeengt wurde. Das öl wurde in heißem Isopropanol aufgenommen und mit

equimolarer heißer isopropanolischer Oxalsäurelösung versetzt. Nach dem Abkühlen erhielt man 85 g 4-(2-(N-Diethylamino)ethoxi)-2,6-dimethyl-acetophenon.

7,65 g (0,029 mol) der Aminbase des obigen Produkts und 6,98 g (0,058 mol) o-Tolylaldehyd wurden in 100 ml Methanol gelöst. Man gab noch 11,6 g 50 %ige Natronlauge zu, kochte alles 2 h unter Rückfluß und ließ über Nacht bei RT rühren. Das Reaktionsgemisch wurde i. Vak. eingeengt, mit Wasser verdünnt und mit Methylenchlorid extrahiert. Die organische Phase wurde noch mit Wasser gewaschen, getrocknet und i. Vak. eingeengt. Den so erhaltenen Rückstand löste man in wenig heißem Ethanol und versetzte mit equimolarer ethanolischer Fumarsäurelösung. Man erhielt 9,5 g des 4'-(2-(N,N-Diethylamino)ethoxi)-2',6'-dimethyl-2-methyl-chalkonfumarats.
Schmp. 132-134 °C
Analog Beispiel 11 wurden hergestellt:

12. 3',5'-Dimethyl-4'-(2-(1-pyrrolidinyl)ethoxi)-2-ethyl-chalkon-fumarat
Schmp. 138-140 °C
13. 3',5'-Dimethyl-4'-(2-(1-pyrrolidinyl)ethoxi)-2-methoxi-chalkon-fumarat
Schmp. 141-145 °C
14. 3',5'-Dimethyl-4'-(2-(1-pyrrolidinyl)ethoxi)-3-methoxi-chalkon-hydrochlorid
Schmp. 169-173 °C
15. 3',5'-Dimethyl-4'-(2-(1-pyrrolidinyl)ethoxyi)-4-methoxi-2-methylchalkon-oxalat
Schmp. 167 °C
16. 2', 6'-Dimethyl-4'(2-(1-piperidinyl)ethoxi)-2-methoxichalkon-fumarat
Schmp. 127-131 °C
17. 3',5'-Dimethyl-4'(2-(1-piperidinyl)ethoxi)-2-methoxichalkon-hydrochlorid
Schmp. 201-203 °C
18. 3',5'-Dimethyl-4'(2-(1-morpholinyl)ethoxi)2-ethylchalkon-hydrochlorid
Schmp. 193-196 °C
19. 2',6'-Dimethyl-4'(2-(1-pyrrolidinyl)ethoxi)2-ethylchalkon-hydrochlorid
Schmp. 153-155 °C
20. 3',5'-Dimethyl-4'(2-(1-piperidinyl)ethoxi)4-methoxi-2-methyl-chalkon-fumarat
Schmp. 163-166 °C

Beispiel 21

4'(3-Diethylamino-propoxi)-2',6'-dimethyl-2-methylchalkon-hydrochlorid

12,0 g (73 mmol) 2,6-Dimethyl-4-hydroxiacetophenon und 17,6 g (146 mmol) 2-Tolylaldehyd wurden in 150 ml Methanol gelöst, mit 29 g 50 %iger Natronlauge (365 mmol) versetzt und 2 h auf Rückfluß erwärmt. Man ließ über Nacht bei RT rühren und engte i.Vak. ein. Der Rückstand wurde in verdünnter Salzsäure aufgenommen und mit Methylenchlorid extrahiert. Die organische Phase wurde getrocknet und i. Vak. eingeengt. Das so erhaltene Produkt wurde aus Petrolether umkristallisiert. Man erhielt 14 g 2',6'-Dimethyl-4'-hydroxi-2-methylchalkon.

4,0 g (15 mmol) des obigen Produkts, 2,25 g (15 mmol) N-3-Chlorpropyl-diethylamin, 4,1 g (30 mmol) Kaliumkarbonat und 0,2 g 18-krone-6 wurden in 50 ml Ethylmethylketon 4 h unter Rückfluß gekocht. Das Karbonat wurde abfiltriert und das Filtrat i. Vak. eingeengt. Der Rückstand wurde in verdünnter Salzsäure aufgenommen und mit Ether gewaschen. Die wäßrige Phase wurde mit verdünnter Natronlauge alkalisch eingestellt und mit Methylenchlorid extrahiert. Die organische Phase wurde getrocknet und mit überschüssiger etherischer Chlorwasserstoff-Lösung versetzt. Man erhielt 4,7 g des Produkts als Hydrochlorid.
Schmp. 162-165 °C
Analog Beispiel 21 wurden hergestellt:

22. 2′,6′-Dimethyl-4′(3-(1-pyrrolidinyl)propoxi)-2-methylchalkon-hydrochlorid
Schmp. 217-218 °C
23. 2′,6′-Dimethyl-4′(2-(1-piperidinyl)ethoxi)-2-methylchalkon-hydrochlorid
Schmp. 193-194 °C
24. 3′,5′-Dimethyl-4′(2-(1-pyrrolidinyl)ethoxi)-4-methoxi-chalkon-fumarat
Schmp. 185-186 °C

Beispiel 25

3,5-Dimethyl-4(2-(1-pyrrolidinyl)ethoxi)-3′-(2-tolyl)-propiophenon-fumarat

8.75 g (110 mmol) des 3′,5′-Dimethyl-4′(-2-(1-pyrrolidinyl)-ethoxi)-2-methylchalkon-tosylats wurden in 100 ml Methanol gelöst, mit 1 g Palladium/Kohle (10 %ig) versetzt und bis zum equimolaren Wasserstoffverbrauch hydriert. Der Katalysator wurde abfiltriert, das Filtrat i. Vak. eingeengt. Das Produkt wurde mit Natronlauge behandelt und mit Fumarsäure gefällt. Man erhielt 4,5 g des o.g. Fumarats.
Schmp. 108-110 °C

Analog Beispiel 25 wurden hergestellt:

26. 2,6-Dimethyl-4(2-(1-pyrrolidinyl)ethoxi)-3′-(2-tolyl)-propiophenon-hydrochlorid
Schmp. 124-126 °C
27. 3,5-Dimethyl-3′-(2-methoxiphenyl)-4(2-(1-piperidinyl)ethoxi)-propiophenon-fumarat
Schmp. 121-123 °C
28. 3,5-Dimethyl-4(2-(1-piperidinyl)ethoxi)-3′-(2-tolyl)-propiophenonfumarat
Schmp. 117-120 °C
29. 4(2-(1-Diethylamino)ethoxi)-3,5-dimethyl-3′-(2-tolyl)-propiophenon-fumarat
Schmp. 83-86 °C
30. 3,5-Dimethyl-4(3-(1-pyrrolidinyl)propoxi)-3′-(2-tolyl)-propiophenon-fumarat
Schmp. 186-187 °C
31. 3,5-Dimethyl-3′-(2-ethylphenyl)-4(2-(1-pyrrolidinyl)ethoxi)-propiophenon-fumarat
Schmp. 96-98 °C
32. 3,5-Dimethyl-3′(2-ethylphenyl)-4(2-(1-piperidinyl)ethoxi)-propiophenon-fumarat
Schmp. 126-129 °C
33. 3,5-Dimethyl-3′-(2-methoxiphenyl)-4(2-(1-pyrrolidinyl)-ethoxi)-propiophenon-fumarat
Schmp. 102-106 °C
34. 3,5-Dimethyl-3′-(4-methoxiphenyl)-4(2-(1-pyrrolidinyl)-ethoxi)-propiophenon-fumarat
Schmp. 135-138 °C
35. 3,5-Dimethyl-3′-(4-methoxiphenyl)-4(2-(1-piperidinyl)-ethoxi)-propiophenon-tosylat
Schmp. 125-128 °C
36. 3,5-Dimethyl-(3′-(4-methoxi-2-methylphenyl)-4(2-(1-pyrrolidinyl)-ethoxi)-propiophenon-hydrochlorid
Schmp. 131-136 °C
37. 3,5-Dimethyl-(3′-(4-methoxi-2-methylphenyl)-4(2-(1-piperidinyl)ethoxi)-propiophenon-fumarat
Öl

## Patentansprüche

**1.** p-Hydroxiphenon-Derivate der allgemeinen Formel I

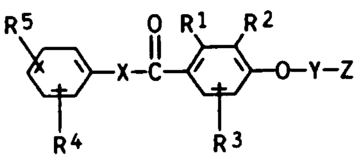

I,

worin
$R^1$, $R^2$ und $R^3$ gleich oder verschieden sind und Methyl, Chlor, Brom sowie Wasserstoff bedeuten, wobei $R^1$, $R^2$ und $R^3$ nicht gleichzeitig H sind, bedeuten,
$R^4$ und $R^5$ gleich oder verschieden sind und H, $C_1$-$C_4$-Alkyl oder $OR^6$ darstellen, wobei $R^4$ und $R^5$ nicht gleichzeitig H sind,
X eine -CH=CH-, $-CH_2CH_2-$ oder $-CH_2$-Brücke ist,
Y eine gerade oder verzweigte, gesättigte oder ungesättigte $C_1$-$C_4$-Brücke darstellt und
Z den Rest $NR^6R^7$ darstellt, wobei $R^6$ und $R^7$ unabhängig voneinander Wasserstoff oder $C_{1-4}$-Alkyl bedeuten, oder Z einen 5- oder 6-gliedrigen Ring mit einem Stickstoffatom in 1-Stellung und ggf. zusätzlich einem zweiten N- oder einem O- oder S-Atom bedeutet, wobei das zweite N-Atom durch $R^7$ substituiert ist,

sowie deren physiologisch verträglichen Salze.

2. p-Hydroxiphenon-Derivate nach Anspruch 1, dadurch gekennzeichnet, daß $R^1$ und $R^2$ $CH_3$-Gruppen in 3,5-Stellung darstellen, wobei $R^3$ = H ist.

3. Arzneimittel zur topischen Anwendung, das neben üblichen Hilfsstoffen als Wirkstoff 0,0001 bis 1 Gew.% eines p-Hydroxiphenon-Derivates nach Anspruch 1 enthält.

4. Arzneimittel zur systemischen Anwendung, das neben üblichen Hilfsstoffen pro Einzeldosis 10 bis 500 mg eines p-Hydroxiphenon-Derivates nach Anspruch 1 enthält.

5. Antiarrhythmikum der Klasse III nach Vaughan-Williams, das neben üblichen Hilfsstoffen eine wirksame Menge eines p-Hydroxiphenon-Derivates nach Anspruch 1 enthält.

## Claims

1. A p-hydroxy phenone derivative of the formula I

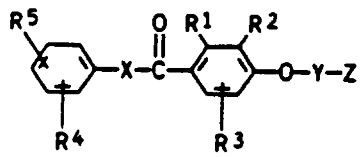

I

where
$R^1$, $R^2$ and $R^3$ are identical or different and are methyl, chlorine, bromine and hydrogen, but $R^1$, $R^2$ and $R^3$ are not all H,
$R^4$ and $R^5$ are identical or different and are H, $C_1$-$C_4$-alkyl, or $OR^6$, but $R^4$ and $R^5$ are not both H,
X is a -CH=CH-, $-CH_2CH_2-$ or $-CH_2-$ bridge,
Y is a straight-chain or branched, saturated or unsaturated $C_1$-$C_4$ bridge, and
Z is $NR^6R^7$, where $R^6$ and $R^7$ are, independently of one another, hydrogen or $C_{1-4}$-alkyl, or Z is a 5- or 6-membered ring with a nitrogen atom in the 1 position and, where appropriate, additionally a second N atom or an O or S atom, the second N atom being substituted by $R^7$,
as well as the physiologically tolerated salts thereof.

2. A p-hydroxy phenone derivative as claimed in claim 1, wherein $R^1$ and $R^2$ are $CH_3$ groups in the 3,5 positions and $R^3$ is H.

3. A drug for topical application which, besides conventional aids, contains as active substance from 0.0001 to 1 % by weight of a p-hydroxy phenone derivative as claimed in claim 1.

4. A drug for systemic administration which, besides conventional aids, contains per single dose from 10 to 500 mg of a p-hydroxy phenone derivative as claimed in claim 1.

5. An antiarrhythmic of Vaughan-Williams class III which, besides conventional aids, contains an effective amount of a p-hydroxy phenone derivative as claimed in claim 1.

## Revendications

**1.** Dérivés de la p-hydroxyphénone de la formule générale I

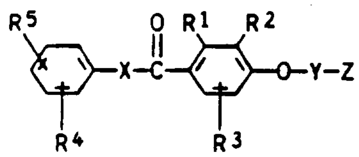

I,

dans laquelle
$R^1$, $R^2$ et $R^3$ sont identiques ou différents et représentent chacun le radical méthyle, l'atome de chlore, l'atome de brome ainsi que l'atome d'hydrogène, mais avec la condition que $R^1$, $R^2$ et $R^3$ ne représentent pas simultanément des atomes d'hydrogène,
$R^4$ et $R^5$ sont identiques ou différents et représentent chacun l'atome d'hydrogène, un radical alkyle en $C_1$-$C_4$ ou le groupe $OR^6$, avec la condition que $R^4$ et $R^5$ ne représentent pas simultanément des atomes d'hydrogène,
X représente un pont $-CH=CH-$, $-CH_2CH_2-$ ou $-CH_2-$,
Y représente un pont en $C_1$-$C_4$, linéaire ou ramifié, saturé ou insaturé et
Z représente le groupe $NR^6R^7$, où $R^6$ et $R^7$ représentent, chacun indépendamment l'un de l'autre, un atome d'hydrogène ou un radical alkyle en $C_1$-$C_4$, ou bien Z représente un noyau pentagonal ou hexagonal avec un atome d'azote en position 1 et, éventuellement complémentairement, un second atome d'azote, ou un atome d'oxygène ou un atome de soufre, où le second atome d'azote est substitué par $R^7$,
ainsi que leurs sels physiologiquement tolérables.

**2.** Dérivés de la p-hydroxyphénone suivant la revendication 1, caractérisés en ce que $R^1$ et $R^2$ représentent des radicaux $CH_3$ en positions 3,5, où $R^3$ représente un atome d'hydrogène.

**3.** Médicament destiné à l'application topique, qui contient, outre les adjuvants usuels, de 0,0001 à 1% en poids d'un dérivé de la p-hydroxyphénone suivant la revendication 1 à titre de principe actif.

**4.** Médicament destiné à l'administration systémique, qui contient, outre les adjuvants usuels, par dose individuelle, 10 à 500 mg d'un dérivé de la p-hydroxyphénone suivant la revendication 1.

**5.** Antiarythmique de la classe III selon Vaughan-Williams qui contient, outre les adjuvants usuels, une proportion efficace d'un dérivé de la p-hydroxyphénone suivant la revendication 1.